# EUROPEAN PATENT SPECIFICATION

(11) **EP 2 765 948 B1**
(45) Date of publication and mention of the grant of the patent: **21.08.2019**
(21) Application number: 12766992.7
(22) Date of filing: 03.10.2012
(51) Int. Cl.: A61C 3/00, A46B 3/22, A46B 9/00, A61Q 11/00, A61K 8/73, A61K 8/24

(54) **ORAL CARE COMPOSITION, APPLICATOR FOR SUCH A COMPOSITION AND METHOD FOR ORAL CARE**
ZAHNÄRZTLICHE ZUSAMMENSETZUNG, INSTRUMENT ZUM VERSTREICHEN DIESER ZUSAMMENSETZUNG UND VERFAHREN ZUR ZAHNÄRZTLICHEN PFLEGE
COMPOSITION POUR SOIN DENTAIRES, INSTRUMENT POUR L'APPLIQUER EN BOUCHE ET PROCÉDÉ POUR SOINS DENTAIRES.

(30) Priority: 05.10.2011 EP 11183998
(43) Date of publication of application: 20.08.2014
(73) Proprietor: Multi Oral BV, 2628 XJ Delft (NL)
(72) Inventor: KOUMANS, Floris, NL-2628 XJ Delft (NL)
(74) Representative: Ringeling, Patricia Louise
(86) International application number: PCT/EP2012/069554
(87) International publication number: WO 2013/050432

(56) References cited:
- EP-A1- 2 072 022
- EP-A1- 2 233 106
- WO-A1-01/34109
- WO-A1-97/26041
- WO-A1-99/47108
- DE-C- 532 700
- DE-U1- 9 007 035
- DE-U1- 20 310 771
- US-A1- 2004 237 233
- US-A1- 2010 297 575

## Description

### Field of the Invention

The present invention relates to a kit for oral care. In particular to a kit for use in a method for treating or preventing gum disease, tooth decay and sensitive teeth.

### Background of the invention

Oral care products are widely used to prevent or treat oral problems. Generally speaking, there are two types of oral care products.

The first group of oral care products comprises products which are primarily designed and formulated to counteract microbial, especially bacterial, problems. These products are used to clean the teeth and to remove food rests and plaque, which promote bacterial growth, from the mouth. Such products are for example described in WO 2007/053581, which describes growth control of oral microorganisms using gallium compounds, and WO2011/023830 which describes antibacterial compositions for oral use. Tooth brushes, tooth floss and tooth paste belong to this category.

The other group of oral care products comprises remineralisation agents which are primarily designed and formulated to counter demineralisation or decalcification of the teeth enamel, which is caused by acid challenges. Remineralising agents belong to this group. These agents typically contain a source of calcium and phosphate ions, for example hydroxyapatite, to replenish the calcium and phosphate which is lost from the tooth enamel in case of demineralisation. Remineralisation agents are for example described in EP 2 289 482, which describes polymeric mineral surface active agents and metal ions.

One disadvantage of existing oral care products is that they tackle either the bacterial problems or the demineralisation problem, and very seldom the combination, although the bacterial problems and demineralisation are related. Bacteria group together to form plaque. In combination with their by-products these bacteria cause demineralisation and tooth erosion. If this is not corrected it may lead to caries and eventually tooth loss. At the same time, acid food and drinks cause tooth erosion. Tooth erosion roughens the tooth enamel and makes is easier for plaque to form on the enamel. The plaque and the disturbance of the periodontal bacterial floral are one of the causes of gingivitis, which is characterised by bleeding gums. Gingivitis may lead to withdrawal of the gums which exposes the dental necks. The dental necks are sensitive, especially to dental caries and this leads to gingivitis again. Apparently, this has not been realised enough before.

Another disadvantage is that the existing oral care products, such as mouth washes and rinses, only allow for short treatment and contact times and they should not be swallowed. Effective oral care treatment, not only requires combined action against demineralisation and against disturbed bacterial flora, but also requires the action to be in the right place for an appropriate length of time. Oral care products with longer contact time exist, for example fluoride treatments, but these are generally not involved in combined action against demineralisation and against disturbed bacterial flora. Furthermore, these oral care products typically require the applicator to be present during the contact time, which severely limits the user's comfort and therefore limit the ease of use.

Document WO 01/34109 discloses dual phase oral compositions providing effective antimicobial activity for reducing plaque and gingivitis. One of the phases of the dual phase composition will contain stannous. The stannous phase comprises a stannous ion source, a fluoride ion source, and a gluconate salt. The dual phase oral compositions known from this document also comprise some polymeric mineral surface active agents, such as polysaccharide.

Document EP2 072 022 discloses an applicator of an oral care liquid for infiltrating enamel lesions. The applicator improves the protection against other areas being contaminated with the liquid. The safety of use is increased. More targeted dosing is also possible.

### Short description of the Figures

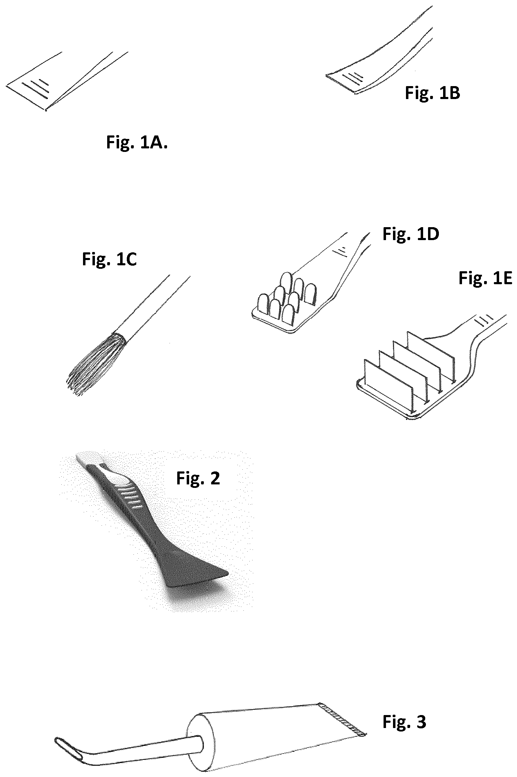

**Fig.** 1 Embodiments of applicator tips for use in the kit according to the invention. **(1A)** A schematic representation of a planar applicator tip which may be used in the method described herein. **(1B)** A schematic representation of concave planar applicator tip. **(1C)** A schematic representation of one embodiment of an applicator tip which becomes a planar sheet when in use. **(1D)** A schematic representation of another embodiment of an applicator tip which becomes a planar sheet when in use. **(IE)** A schematic representation of another embodiment of an applicator tip which becomes a planar sheet when in use.
**Fig. 2** A spatula with flexible tip which can be used for applying an oral care product which is used in a method described herein.
**Fig.3** An applicator integrated with the container which contains the oral care composition. The integrated applicator and container may be part of-a kit according to the invention.

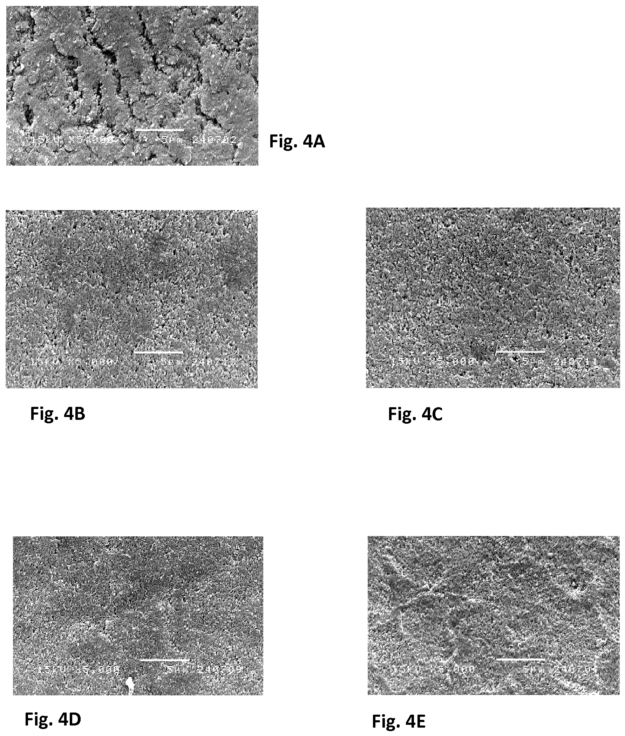

**Fig. 4** Scanning electronmicroscopic pictures of enamel, magnification 5000x. **(4A)** Enamel after drinking a soda drink; and after a subsequent exposure of **(4B)** 30 min, **(4C)** 1 hour, **(4D)** 3 hours, **(4E)** 6 hours to a remineralising oral care composition according to the invention.

### Detailed description

The present invention relates to a kit according to claim 1. The kit may be used in a method as described herein for treating or preventing gum disease, tooth decay or sensitive teeth, wherein the method comprises:
i) applying a non-abrasive oral care composition comprising 0.05-10% v/v of a remineralising agent and 0.05-20% v/v of a bio-adhesive agent to the gum and teeth of an individual using an applicator which is suitable for plastering a layer of the oral care composition on the gum and teeth of the individual,
ii) allowing the oral care composition a contact time with the gum and teeth of the individual of at least 5 minutes.

The method described herein is simple, easy to use and therefore very effective in giving teeth and gums a healthier look and feel and condition. Because it is easy to use, it is very convenient as a self-care method and does not require professional intervention. Regular application of the method will condition the gums, prevent plaque formation, repair tooth erosion, treat bleeding gums, reduce swellings, reinforce the tooth enamel and decrease sensitive teeth. The applicator is preferably removed from the oral cavity directly after application of the oral care composition and is preferably not present during contact time, which adds to the ease of use and to the user's comfort.

According to the method described herein, a non-abrasive oral care composition comprising 0.05-10% v/v of a remineralising agent and 0.05-20% v/v of a bio-adhesive agent is applied to the gum and teeth of an individual and allowed contact with the gum and teeth for at least 5 minutes, preferably for at least 10 minutes, at least 15 minutes, at least 20 minutes, at least 25 minutes or at least 30 minutes, more preferably for at least 60 minutes, even more preferably for at least 2 hours, at least 3 hours or at least 4 hours. Most preferably, the non-abrasive oral care composition is allowed contact with the gum and teeth for at least 5 hours, at least 6 hours, at least 7 hours or at least 8 hours. This can for example be achieved by applying the non-abrasive oral care composition overnight during night rest, preferably after normal night brushing of the teeth just prior to bed time. The applicator which is used to apply the non-abrasive oral care composition is preferably removed from the mouth directly after application of the oral care composition and is preferably not present during contact time. In the present context, 'to allow contact' indicates that the oral care composition is not removed from the mouth purposely, for example by spitting or rinsing, but is allowed to stay in the mouth. Users preferably do not eat or drink for at least 5 minutes, preferably for at least 10 minutes, at least 15 minutes, at least 20 minutes, at least 25 minutes or at least 30 minutes, more preferably for at least 60 minutes, even more preferably for at least 1 hour, at least 2 hours, at least 3 hours or at least 4 hours, yet even more preferably for at least 5 hours, at least 6 hours, at least 7 hours or at least 8 hours after using the oral care composition according to the method.

The method can be used to treat or prevent gum disease, tooth decay or sensitive teeth. Regular application of the method will condition the gums, prevent plaque formation, repair tooth erosion, treat bleeding gums, reduce swellings, reinforce the tooth enamel and decrease sensitive teeth. In the present context, 'gum disease' refers to periodontal diseases. Periodontal diseases include gingivitis, which is an inflammation of the gums, and several different forms of periodontitis, in which the inflammation has spread from the gums to the bones in the mouth. In particular bleeding gums can be treated with the method. In the present context, 'tooth decay' refers to demineralisation of the hard tooth structure, be it surface or subsurface. 'Tooth erosion' refers to a superficial, often mild form of tooth enamel demineralisation which is not localized but affects the general surface of the teeth, and which may eventually lead to tooth decay. Demineralisation may lead to sensitive teeth, especially so when the underlying layer of the teeth, the dentine, becomes exposed. The demineralisation may be due to a variety of causes. It may be due to acid challenges in relation to for example dental plaque or acid food and drinks, such as fruit juices and soft drinks. Alternatively, it may be due to medication and disease which inflict dry mouth problems and problems with saliva production. Saliva is important for the oral flora and for remineralisation. Therefore, chronic decrease of saliva production, e.g. by disease or medication, compromises the oral flora and the natural remineralisation.

The non-abrasive oral care composition comprising 0.05-10% v/v of a remineralising agent and 0.05-20% v/v of a bio-adhesive agent is preferably applied at least twice a week after brushing the teeth. More preferably, the -oral care composition is applied -at least three times a week, at least four times a week or at least five times a week after brushing the teeth. Most preferably,- the oral care composition is applied once a day after brushing the teeth. Preferably, it is applied in the evening before going to bed, in order to allow a long and intense contact time with the oral tissues. For optimal contact, after application the mouth is not rinsed and no drink or food is used for at least 5 minutes, preferably for at least 10 minutes, at least 15 minutes, at least 20 minutes, at least 25 minutes or at least 30 minutes, more preferably for at least 60 minutes, even more preferably for at least 1 hour, at least 2 hours, at least 3 hours or at least 4 hours, yet even more preferably for at least 5 hours, at least 6 hours, at least 7 hours or at least 8 hours. The contact time in the method is longer than the contact time typically applied when brushing with tooth paste (usually 2 minutes at the most) or when using mouth washes (usually less than a minute).

According to the method described herein, the oral care composition may or may not be swallowed after the contact time. Preferably, only food grade ingredients are used- in the oral care composition. In the context of the present invention, "food grade" indicates that something is safe to swallow regularly, without adverse health effects. In one embodiment of the method, a food grade oral care composition is used, the remains of which are swallowed after the contact period.

In the context of the present invention, 'oral care composition' refers to a non-abrasive dental medium possessing curative and prophylactic properties employed in oral hygiene. The oral care composition comprises 0.05-10% v/v of a remineralising agent. It should have a consistency and composition which allows for a contact time of several minutes, preferably of several hours, therefore it comprises 0.05-20% v/v of a bioadhesive, optionally in combination with a thickening agent. The oral care composition preferably has a viscosity between 800 and 40000 cPs, preferably between 1000 and 35 000 cPs, measured at 19-21 degrees Celsius and at a spindle 6 at 12 rpm or spindle 7 at 20 rpm using a Viscometer & Model Brookfield DV-II+ Programmable, to allow a contact time of at least 5 minutes. In particular gels or pastes can suitably be used in the method.

The method is preferably used on teeth which have been cleaned, for example -after brushing, in order to maximise the contact between teeth and gum on the one hand and the non-abrasive oral care composition on the other hand. After brushing the teeth properly, the biofilm or plaque has been sufficiently removed so that the efficacy of the composition is enhanced. The oral care composition is preferably applied to the inside and the outside of the teeth. In the present context, 'teeth' refer to any kind of teeth, including natural teeth, artificial teeth and dentures. The teeth and gum may be of any individual, be it a human being, such as for example an adult or a child, or an animal, such as a cat, a dog or a horse. The skilled person will understand that when animals are treated, the oral care compositions is most suitably applied to the animal's teeth by a human being or by the intervention of a human being.

Application of the non-abrasive oral care composition to the teeth and gums may be in any convenient way which allows the oral care composition to be applied into the pockets in between the teeth and to the area where the gums meet the teeth, which, in the context of the present application, is also referred to as plastering-, which provides an overall layer or film over the gum and teeth. Application of the oral care composition is typically over the general surface of the teeth and gums -and not at one or two specific spots. Preferably, it is applied to both the inside and the outside of the teeth, into the pockets in between the teeth and to the area where the gums meet the teeth. In a preferred embodiment, the non-abrasive oral care composition is plastered on teeth and gums using an applicator.

In a preferred embodiment, the method for treating or preventing tooth erosion comprises:
i) applying a non-abrasive oral care composition comprising 0.05-10% v/v of a remineralising agent and 0.05-20% v/v of a bio-adhesive agent to the gum and teeth of an individual using an applicator which is suitable for plastering a layer of the oral care composition on the gum and teeth of the individual,
ii) allowing the oral care composition a contact time with the gum and teeth of the individual of at least 5 minutes.

The oral care composition is a non-abrasive dental medium possessing curative and prophylactic properties and is employed in oral hygiene. Preferably, only food grade ingredients are used in the oral care composition. This allows for the oral care composition to be swallowed regularly without adverse health effects. The oral care composition comprises 0.05-10% v/v of a remineralising agent. It should have a consistency and composition which allows for a contact time of several minutes, preferably of several hours, therefore it comprises 0.05-20% v/v of a bioadhesive, optionally in combination with thickening agents. The oral care composition, preferably has a viscosity between 800 and 40 000 cPs, more preferably between 1000 and 35000 cPs, measured at 19-21 degrees Celsius and a spindle 6 at 12 rpm or a spindle 7 at 20 rpm using a Viscometer & Model Brookfield DV-II+ Programmable, to allow a contact time of at least 5 minutes. In particular gels or paste can suitably be used in the method.

In one embodiment, the non-abrasive oral care composition comprising 0.05-10% v/v of a remineralising agent and 0.05-20% v/v of a bio-adhesive agent is for use in treating or preventing gum disease, tooth decay or sensitive teeth, wherein the treatment or prevention comprises:
i) applying the oral care composition to the gum and teeth of an individual using an applicator which is suitable for plastering a layer of the oral care composition on the gum and teeth of the individual;
ii) allowing the oral care composition a contact time with the gum and teeth of at least 5 minutes.

In another embodiment, the non-abrasive oral care composition comprising 0.05-10% v/v of a remineralising agent and 0.05-20% v/v of a bio-adhesive agent is for use in a method for treating or preventing tooth erosion, wherein the method comprises:
i) applying the oral care composition to the gum and teeth of an individual using an applicator which is suitable for plastering a layer of the oral care composition on the gum and teeth of the individual;
ii) allowing the oral care composition a contact time with the gum and teeth of at least 5 minutes.

In yet another embodiment, the kit comprises a food grade non-abrasive oral care composition comprising 0.05-10% v/v of a remineralising agent- and 0.05-20% v/v of a bio-adhesive agent for use in a method for treating or preventing gum disease, tooth decay or sensitive teeth, or in a method for preventing or treating tooth erosion, wherein the method comprises:
i) applying the oral care composition to the gum and teeth of an individual using an applicator which is suitable for plastering a layer of the oral care composition on the gum and teeth of the individual;
ii) allowing the oral care composition a contact time with the gum and teeth of at least 5 minutes, and ;
iii) swallowing the remaining oral care composition by the individual.

The non-abrasive oral care composition for use in the method comprises 0.05-10% v/v of a remineralising agent. Remineralising agents are known in the art and include fluoro, calcium or phosphate ion containing compositions, such as for example sodium fluorite, amorphous calcium phosphate, Galla Chinensis and apatites, such as hydroxyapatite, fluoroapatite and chloroapatite. Preferably, food grade remineralising agents are used. The remineralising agent must be present in the oral care composition in an effective amount, i.e. in an amount which ensures bio-available amounts during use in the mouth. The effective amount will depend on the remineralising agent used. One or more remineralising agents may be combined for optimal effect. For many remineralising agents this will be in a total concentration of 0.05 - 10% v/v, preferably in a concentration of 0.1 - 5% v/v, more preferably in a concentration of 0.1 - 1% v/v by total volume of the oral care composition.

To allow a contact time of at least 5 minutes between teeth and gum on the one hand and the oral care composition on the other hand, the oral care composition for use in the method also comprises 0.05-20% v/v of a bioadhesive. Any bioadhesive known in the art may be included in the oral composition. Suitable examples include polysaccharides, such as celluloses and pectins; carbomers, macrogols (polyethylene glycols), Amigel -and natural gums such as Karaya gum, xanthan gum, carrageenan, gum arabic, scerotium gum and gum tragacanth. Preferably, food grade bioadhesives are used. The one or more bioadhesives are present in an effective amount, i.e. in an amount which allows attachment of the oral care composition to the teeth and gum and which allows a contact time of at least 5 minutes. The effective amount will very much depend on the nature of the bioadhesive. In many cases, an effective amount will be in a total concentration of 0.05-20% v/v, preferably, in a concentration of 0.1-10% v/v more preferably in a concentration of 0.5-5% v/v, by total volume of the oral care composition. In one embodiment, the oral care composition comprises 1.5% v/v xanthan gum as a bioadhesive. In another embodiment, the oral care composition comprises 3% v/v xanthan gum as a bioadhesive. The oral care composition is preferably formulated as a gel or a paste.

The non-abrasive oral care composition for use in the method preferably also comprises an antimicrobial agent, in particular an anti-plaque agent. In the present context, the terms 'anti-plaque agent', 'antimicrobial agent' and 'microbial control agent' are used interchangeably and refer to a compound which inhibits, controls or kills microorganisms associated with dental plaque formation, gingivitis or periodontitis and which is acceptable for oral use. Such agents are known in the art and include disinfectants and bacterial adhesion blockers, such as chlorhexidin, hexetidine, delmopinol, fluorides, such as sodium fluoride and stannous fluoride; Miswak (obtainable from the Salvadore persica tree), triclosan, essential oils, magnesium ascorbyl phosphate, sodium ascorbyl phosphate and phenolic compounds, such as hydroxy chavicol. Preferably, food grade microbial control agents are used. The anti-plaque agent is present in an effective amount, i.e. in an amount which inhibits, controls or kills cariogenic or periodontopathogenic microorganisms in the oral cavity. One or more antimicrobial agents may be combined for optimal effect. The effective amount will very much depend on the nature of the antimicrobial agent. For example, an effective amount for Miswak will be a concentration of 1-15% v/v, whereas an effective amount for sodium ascorbyl phosphate will be a concentration of 0.1-10% v/v, by total volume of the oral care composition. In one embodiment, two or more antimicrobial agents are combined. In one embodiment, the oral care composition comprises 1-2% v/v Miswak and 0.5-1% v/v sodium ascorbyl phosphate. In another preferred embodiment, a selective antimicrobial agent is used, i.e. one which selectively affects, in particular blocks, the adhesion of the cariogenic and periodontopathogenic microorganisms, be it bacteria, fungi or yeasts, but does not affect the biological equilibrium in the mouth. Preferably, it affects *Streptococcus mutans, Streptococcus sobrinus, Staphylococcus aureus, Actinobacillus actinomycetemcomitans, Staphylococcus epidermidis, Pseudomonas aeruginosa, Klebsiella pneumonia, Proteus mirabilis, Proteus vulgaris, Candida albicans* and *Aspergillus fumigatus.* Preferably, it does not affect *Streptococcus salivarius.* Such compositions are known in the art. A suitable example is the anti-microbial composition described in EP 1 461 361. This composition comprises polysaccharides which are derivable from *Aloe vera.*

In one embodiment, the non-abrasive oral care composition for use in the method also comprises a whitening agent. Suitable whitening agents for oral care are known in the art. Preferably, a food grade whitening agent is used. Whitening agents which do not require scrubbing to have their whitening effect are preferred, e.g. peroxides, such as hydrogen peroxide or carbamide peroxide. Depending on the whitening agent used, the whitening agent may be present in the oral care composition in a concentration of -2-50% v/v.

The oral care composition which is used in the method must have a pH which does not adversely affect the teeth and should not have a negative influence on the tissue or the natural immune response. A suitable pH is typically between about pH 6.0 and about pH 7.6, preferably between about pH 6.2 and about pH 7.4. The pH may conveniently be adjusted using an orally compatible buffering agent, i.e. one which does not harm the teeth and is not harmful when swallowed in the composition used. Further suitable ingredients for an oral care composition to be used in the method are sweeteners, preservatives, anti-oxidants and flavouring agents. Any sweetener which is acceptable for oral use may be used. Preferably, a food grade sweetener is used. Suitable examples are known in the art and include both natural and artificial sweeteners, such as acesulfame potassium, aspartame, Charm Sweet Artificial Sweetener, cyclamate, fructose, glucose, neohesperidine dihydrochalcone, neotame, glycerine, inulin, maltitol, mannitol, levulose, saccharin, sorbitol, stevia, sucralose, tagatose, thaumatine, trehalose and xylitol. Any flavouring agent which is acceptable for oral use may be used. Suitable examples are known in the art and include menthol, fruit flavours, herbal derivatives, thymol and anise oil. Preferably, a food grade flavouring agent is used. Any anti-oxidant which is acceptable for oral use may be used. Suitable examples are known in the art and include sodium ascorbyl phosphate. Preferably, a food grade anti-oxidant is used. Any preservative which is acceptable for oral use may be used. Suitable examples are known in the art and typically include preservatives suitable for foodstuffs or preservatives which do not have harmful effects when swallowed in the composition used and include caprylyl glycol, sodium benzoate, potassium sorbate, parabens, decalact, sodium caproyl, sodium lauroyl, lactyl lactate, anisic acid, levulinic acid. The skilled person will understand that many of the mentioned ingredients may have multiple actions and may be used for multiple purposes. For example, an antimicrobial agent may act simultaneously as a flavouring agent and as a bioadhesive.

The non-abrasive oral care composition for use in the method does not contain, or only in negelectible amounts, such as less than 5% w/w abrasive agents, such as phosphates, carbonates, salts, zeolites, alumina and silica. Preferably, the non-abrasive oral care composition for use in the method comprises less than 4% w/w, 3% w/w, 2% w/w or 1% w/w abrasive agents. These abrasive agents are typically found in toothpaste, where they facilitate the removal of bacterial plaque. The non-abrasive oral care composition is used to condition the gums, prevent plaque formation, repair tooth erosion, treat bleeding gums, reduce swellings, reinforce the tooth enamel and decrease sensitive teeth. It is not used for cleansing, scrubbing or otherwise actively and physically removing bacterial plaque and food debris. There is no rubbing, scrubbing or abrasing in the method. Therefore, if an abrasive agent is present in the non-abrasive oral care composition it typically has a non-abrasive function. In one embodiment, hydroxyapatite is present in the non-abrasive oral care composition, but it is not used as an abrasive agent, but as a remineralising agent.

The non-abrasive oral care composition may be applied to the teeth and gums in any convenient way, as long as it is applied into the pockets in between the teeth and to the area where the gums meet the teeth. Preferably, it is applied as if it is plastered on teeth and gums. Preferably, it is applied to both the inside and the outside of the teeth, into the pockets in between the teeth and to the area where the gums meet the teeth. This can conveniently be achieved using an applicator.

The applicator for use in the method described herein is suitable for plastering a layer of an oral care composition on the gum and teeth of an individual. Thereto, the applicator comprises a tip which allows an oral care composition for use in the method to be plastered over the gum and teeth of an individual.

In one embodiment, this is achieved by using a tip which has the form a planar sheet, like the working end of a plaster tool (See Fig. 1A). In this way, the oral care composition may indeed be applied as a layer, coating or a film all over the gum and teeth of an individual, including the pockets in between the teeth and the area where the gums meet the teeth. The substantially planar sheet may be concave to increase the loading capacity of the tip and to conveniently be used for both the inside (lingual) and the outside (buccal or labial) of the teeth (See Fig. 1B). The skilled person will be able to find the best concave form which allows for effective plastering and with minimal waste of product by routine optimisation.

In another embodiment, a tip is used which forms a substantially planar sheet when in use. This can be achieved in many different ways. In one embodiment, this is achieved by using a tip which comprises multiple flexible protrudings . The protrudings may be perpendicular or in the same plane as the base to which they are attached. In one embodiment, the protrudings are of elongated form, like the bristles in a brush (See Fig. 1C). In use, the protrudings of elongated form are bent and form a subtantially planar sheet, which is suitable for plastering a layer, coating or film all over the gum and teeth of an individual, including the pockets in between the teeth and the area where the gums meet the teeth. Preferably, there are between 5 and 3000 flexible protrudings of elongated form per square cm of the tip. The diameter of each protruding of elongated form is preferably between 0.1 and 3 mm. The protrudings of elongated form may be made from any suitable material, including hair, foam, plastic, rubber, silicon. In one embodiment, the applicator in the kit according to the invention is a soft brush, looking like a tooth brush, comprising between 300-3000 soft flexible plastic hairs per square centimeters. The hairs stand wide enough apart and are flexible enough to form a planar sheet when in use. In another embodiment, the protrudings are flexible parallel sheets which in use become overlapping slats, like Venetian blinds (See Fig. 1D and 1E). In one embodiment, there are between 3 and 15 flexible parallel sheets per square cm of the tip. The length of each parallel sheet is between 2 and 8 mm. The parallel sheets may be made from any suitable material-, including hair, foam, plastic, rubber and silicon.

The tip is made from non-absorbent, non-porous flexible material, for example from elastomer material, in order to provide the layer, coating or a film all over the gums and teeth of an individual, including the pockets in between the teeth and the area where the gums meet the teeth. Any non-absorbent, non-porous flexible material which can be formed into a planar sheet, or which can be designed to form a planar sheet when in use, is suitable.

The tip may have any suitable form. In one embodiment, the tip is a planar sheet in the form of a trapezium. If the tip is supported by a finger or a grip, preferably, the shortest parallel -side of the trapezium is supported. This results in a tip which becomes wider at its distal end, which is very convenient for -plastering a layer, coating or film all over the gums and teeth of an individual. In another embodiment, the tip is a slightly concave planar sheet in the form of a trapezium. In yet another embodiment, the tip is in the form of a finger for use as a finger glove with bristles. The length of the tip is preferably between 4 and 50 mm, as to comfortably be used in the mouth of an individual person or animal.

Based on the above-mentioned examples, a skilled person will be able to think of other alternatives tip designs which allow an oral care composition for use in the method to be plastered over the gum and teeth of an individual, including the pockets in between the teeth and the area where the gums meet the teeth, preferably at both the inside (lingual/palatal) and the outside (facial) of the teeth. Any design which results in a planar sheet, as such or when in use, is suitable.

In one embodiment, the applicator -comprises a tip which, in use, is supported and directed by a finger. In another embodiment, the applicator comprises a tip and also comprises a grip. The grip is preferably adjacent to the tip. The grip is used to hold the applicator and to support the tip. Grip and tip may be permanently or detachably connected. The grip is preferably held by the user at the proximal end, while the tip is preferably permanently or detachably connected to the distal end of the grip. Any applicator with a grip and a tip known in the art which can be used to apply a layer, coating or a film all over the gum and teeth of an individual, including the pockets in between the teeth and the area where the gums meet the teeth, preferably at both the inside (lingual/palatal) and the outside(facial) of the teeth, may be used. The grip is preferably of elongated form. In one embodiment, the applicator grip has a length:width ratio of between 12: 1 and 8:1. Preferably, the applicator grip has a length:width ratio of about 10:1. More preferably, the applicator grip has a length:width ratio of 10:1. Any of the tip embodiments described above may be combined with a grip.

In one embodiment, the applicator which comprises a grip and a tip is in the form of a spatula. (See Fig. 2). The grip makes it easier to force the oral care composition into the pockets in between the teeth and to the area where the gums meet the teeth.

In another embodiment, the applicator comprises a grip and a tip and has the form and dimensions of a toothbrush. The skilled person will understand that the applicator is meant to apply something, in particular the oral care composition, and is not an oral cleanser, like a toothbrush, which is used to remove something, viz. dirt, food rest, debris, plaque and bacteria. Although the action is different from a toothbrush, the applicator may have the form and dimensions of a little toothbrush for ease of use, although the tip, which is used to apply the oral care composition, will be different, as described above.

The applicators described may be used in methods described herein. In one embodiment, the applicator described is used in a method for treating or preventing gum disease, tooth decay or sensitive teeth, wherein the method comprises:
i) plastering a non-abrasive oral composition which comprises 0.05-10% v/v of a remineralising agent and 0.05-20% v/v of a bio-adhesive agent to the gum and teeth of an individual using the applicator;
ii) allowing the oral composition a contact time with the gum and teeth of at least 5 minutes.

In another embodiment, the applicators described is used in a method for treating tooth erosion, wherein the method comprises:
i) plastering a non-abrasive oral composition which comprises 0.05-10% v/v of a remineralising agent and 0.05-20% v/v of a bio-adhesive agent to the gum and teeth of an individual using the applicator;
ii) allowing the oral composition a contact time with the gum and teeth of at least 5 minutes. The applicator is preferably not present in the oral cavity after application of the oral care composition. The applicator is also preferably not present during contact time. Therefore, the applicator may be removed from the mouth directly after application of the oral care composition, which adds to the ease of use and to the user's comfort.

The applicator must conveniently be used in the mouth and is therefore preferably 7-15 cm long. In one embodiment, an applicator of about 12 to 13 cm long is used. In another embodiment, an applicator of about 10 to 11 cm long is used. The applicator is made from plastic or any type of elastomer, such as any flexible plastic or rubber. The oral care composition is preferably applied directly after brushing the teeth. The oral care composition is applied to the tip of the applicator. One or more applications may be necessary to have enough oral care composition to cover all gum and teeth of an individual, including the pockets in between the teeth and the area where the gums meet the teeth, preferably at both the inside (lingual/palatal) and the outside(facial) of the teeth. In one embodiment, a total of about 0.8 gram to about 1.5 gram of oral care composition is enough to cover all the teeth properly.

In a preferred embodiment, the applicator is a plastic or rubber spatula with a grip and flexible tip, which is designed in such a way that one side of the spatula tip is very suitable for application of the oral composition to the outside of the teeth and the other side of the spatula tip makes application on the inside of the teeth easy. A suitable example of such an applicator is depicted in Fig. 2.

The applicator is preferably used to apply an oral care composition which comprises a remineralising agent, an antimicrobial agent and a bioadhesive agent, in an effective amount. The applicator is preferably used to apply an oral care composition after the gum and teeth are cleaned from bacterial biofilm or plaque. The applicator is preferably not present in the oral cavity after application of the oral care composition. The applicator is also preferably not present during contact time. Therefore, the applicator is preferably removed from the mouth directly after application of the oral care composition.

The present invention relates to a kit according to claim 1. The above-mentioned embodiments and preferred embodiments of the oral care composition and the applicator are also applicable to the applicator and the oral care composition in the kit.

In one embodiment, the kit comprises an applicator which comprises a tip which is in the form of a planar sheet made from elastomeric material and a non-abrasive oral care composition comprising 0.05-10% v/v of a remineralising agent and 0.05-20% v/v of a bio-adhesive agent, based on the total volume of the oral care composition.

In another embodiment, the kit comprises an applicator which comprises a tip which is in the form of a planar sheet for applying the oral care composition and has a trapezium form and a non-abrasive oral care composition comprising 0.05-10% v/v of a remineralising agent and 0.05-20% v/v of a bio-adhesive agent, based on the total volume of the oral care composition.

In another embodiment, the kit comprises a spatula type applicator which comprises an elastomeric tip in the form of a planar sheet for applying the oral care composition and wherein the length:width of the applicator is about 10:1 and a non-abrasive oral care composition comprising 0.05-10% v/v of a remineralising agent and 0.05-20% v/v of a bio-adhesive agent, based on the total volume of the oral care composition.

In another embodiment, the kit comprises a spatula type applicator which comprises an elastomeric tip in the form of a concave sheet for applying the oral care composition and wherein the length:width of the grip is about 10:1 and a non-abrasive oral care composition comprising 0.05-10% v/v of a remineralising agent and 0.05-20% v/v of a bio-adhesive agent, based on the total volume of the oral care composition.

In another embodiment, the kit comprises a spatula type applicator which comprises an elastomeric tip in the form of a planar sheet in trapezium form, which is optionally slightly concave, for applying the oral care composition and wherein the length:width of the grip is about 10:1 and a non-abrasive oral care composition comprising 0.05-10% v/v of a remineralising agent and 0.05-20% v/v of a bio-adhesive agent, based on the total volume of the oral care composition.

In another embodiment, the kit comprises an applicator which comprises a tip in the form of a brush head having between 5 and 3000 flexible protrudings per square centimeter and a non-abrasive oral care composition comprising 0.05-10% v/v of a remineralising agent and 0.05-20% v/v of a bio-adhesive agent, based on the total volume of the oral care composition.

In another embodiment, the kit comprises an applicator which comprises a tip having between 300 and 3000 soft flexible protrudings per square centimeter and a non-abrasive oral care composition comprising 0.05-10% v/v of a remineralising agent and 0.05-20% v/v of a bio-adhesive agent, based on the total volume of the oral care composition.

In another embodiment, the kit comprises an applicator which comprises a tip in the form of a brush head having between 300 and 3000 flexible protrudings per square centimeter, wherein the protrudings are from elastomer material and a non-abrasive food grade oral care composition comprising 0.05-10% v/v of a remineralising agent and 0.05-20% v/v of a bio-adhesive agent, based on the total volume of the oral care composition, wherein the viscosity of the non-abrasive oral care composition is between 800 and 40 000 cPs as measured by a Brookfield Viscometer, spindle 6 at 12 rpm or a spindle 7 at 20 rpm at about 20 degrees C.

In another embodiment, the kit comprises an applicator which comprises a tip in the form of elastomeric parallel sheets which in use become overlapping slats, like Venetian blinds and a non-abrasive oral care composition comprising 0.05-10% v/v of a remineralising agent and 0.05-20% v/v of a bio-adhesive agent, based on the total volume of the oral care composition.

In another embodiment, the kit comprises an applicator which comprises an elastomeric tip in the form of a substantially planar sheet -which is in the form of a trapezium and a non-abrasive food grade oral care composition comprising 0.05-10% v/v of a remineralising agent -and 0.05-20% v/v of a bio-adhesive agent, based on the total volume of the oral care composition.

In another embodiment, the non-abrasive oral care composition in the kit is a food grade gel or paste.

In another embodiment, the applicator in the kit is of elongated form and comprises at one end a tip which is suitable for plastering a layer of the oral care composition on the gum and teeth of the individual and wherein the wherein the non-abrasive oral care composition has a viscosity of between 800 and 40000 cPs as measured by a Brookfield Viscometer, spindle 6 at 12 rpm or a spindle 7 at 20 rpm at about 20 degrees C .

In another embodiment, the non-abrasive oral care composition in the kit is food grade and further comprises 0.5-50 % v/v of a microbial control agent.

In another embodiment, the non-abrasive oral care composition in the kit is food grade and further comprises 0.5-50% v/v of a microbial control agent, based on the total volume of the oral care composition and 2-50% v/v of a teeth whitening agent.

The kit may comprise directions for use. For example stating that the applicator in the kit is for applying the oral care composition into the pockets in between the teeth and to the area where the gums meet the teeth; and that the applicator is preferably not present in the oral cavity after application of the oral care composition; that the applicator is also preferably not present during contact time and that the applicator is preferably removed from the oral cavity directly after application of the oral care composition.

In one embodiment, the kit comprises both the applicator and the oral care composition, in separate packagings or containers. In one embodiment, the oral care composition is present in the kit in one packaging holding several dosage volumes, for example in a 50 ml container. In another embodiment of the invention, the oral care composition is present in the kit in single dose units, for example in 1-2 ml containers . In one embodiment, the kit comprises five 1-2 ml tubes of the non-abrasive oral care composition together with a suitable applicator. The container comprising the oral care composition is preferably a bottle, flacon or tube, more preferably a tube. The container may comprises a pump mechanism to dispense the oral care composition. In another embodiment, the container is pressurized to allow easy exit of the oral care composition as soon as pressure is released.

The kit may comprise (i) an applicator and (ii) a container comprising the non-abrasive oral care composition, which are meant to be detachably connected for use the method. In one embodiment, the kit comprises an applicator with a hollow grip into which a container comprising the non-abrasive oral care composition can be retained, in part or completely. The oral care composition is then dispensed from its container into the tip of the applicator while retained in the grip of the applicator, on the gum and teeth of an individual. In yet another embodiment, the applicator comprises a tip which can be screwed upon the container comprising the non-abrasive oral care composition. The oral care composition is then dispensed from its container into the tip of the applicator and from there on the gum and teeth of an individual.

In another embodiment, the kit comprises an applicator and a container comprising the non-abrasive oral care composition which are integrated for use in the method according to the invention (Fig. 3). The applicator may be integrated with a container which is pressurized. The oral care composition will easily flow from the container when pressure is released.

In yet another embodiment, a kit according to the invention is formed by (i) a packaging comprising an applicator suitable for plastering over the gum and teeth of an individual, and (ii) a packaging comprising a remineralising agent and a bioadhesive agent, formulated as a gel or paste, which are offered together.

## Claims

1. A kit comprising (i) a non-abrasive oral care composition in the form of a gel or paste comprising 0.05-10% v/v of a remineralizing agent and 0.05-20% v/v of a bioadhesive agent based on the total volume of the oral care composition and (ii) an applicator for applying the non-abrasive oral care composition in a layer on the gum and teeth of the individual, the applicator comprising at one end a tip made from non-absorbent, non-porous flexible material in the form of a planar sheet or forming a planar sheet when in use.

2. A kit according to claim 1, wherein the tip of the applicator is in the form of a trapezium.

3. A kit according to claim 1 or 2, wherein the tip of the applicator is having between 3 and 3000 flexible protrudings per square cm.

4. A kit according to claim 3 wherein the flexible protrudings are in the form of parallel sheets which in use become overlapping slats and form a planar sheet.

5. A kit according to claim 3, wherein the protrudings are of elongated form, like bristles, which in use form a planar sheet.

6. A kit according to claims 2 to 5 wherein the applicator further comprises, adjacent to the tip, a grip which, in use, is to be held by a user.

7. A kit according to claim 6 whereby the grip is of elongated form.

8. A kit according to claims 1 to 7 wherein the non-abrasive oral care composition further comprises a microbial control agent.

9. A kit according to claims 1 to 8, wherein the non-abrasive oral care composition is safe to swallow.

## Patentansprüche

1. Kit, umfassend (i) eine nicht-abrasive Mundpflegezusammensetzung in Form eines Gels oder einer Paste, umfassend 0,05-10 Vol.% eines Remineralisierungsmittels und 0,05-20 Vol.% eines bioadhäsiven Mittels, bezogen auf das Gesamtvolumen der Mundpflegezusammensetzung, und (ii) einen Applikator zum Auftragen der nicht-abrasiven Mundpflegezusammensetzung in einer Schicht auf das Zahnfleisch und die Zähne des Individuums, wobei der Applikator an einem Ende eine Spitze aus nicht-absorbierendem, nicht-porösem, flexiblem Material in Form eines ebenen Blatts umfasst oder bei Verwendung ein ebenes Blatt bildet.

2. Kit nach Anspruch 1, wobei die Spitze des Applikators in Form eines Trapezes vorliegt.

3. Kit nach Anspruch 1 oder 2, wobei die Spitze des Applikators zwischen 3 und 3000 flexible Vorsprünge pro Quadratzentimeter aufweist.

4. Kit nach Anspruch 3, wobei die flexiblen Vorsprünge in Form von parallelen Blättern vorliegen, die im Gebrauch zu überlappenden Lamellen werden und ein ebenes Blatt bilden.

5. Kit nach Anspruch 3, wobei die Vorsprünge von länglicher Form sind, wie Borsten, die im Gebrauch ein ebenes Blatt bilden.

6. Kit nach den Ansprüchen 2 bis 5, wobei der Applikator ferner angrenzend an die Spitze einen Griff umfasst, der im Gebrauch von einem Benutzer gehalten werden soll.

7. Kit nach Anspruch 6, wobei der Griff von länglicher Form ist.

8. Kit nach den Ansprüchen 1 bis 7, wobei die nicht-abrasive Mundpflegezusammensetzung ferner ein mikrobielles Kontrollmittel umfasst.

9. Kit nach den Ansprüchen 1 bis 8, wobei die nicht-abrasive Mundpflegezusammensetzung gefahrlos zu verschlucken ist.

## Revendications

1. Kit comprenant (i) une composition de traitement oral non-abrasive sous la forme d'un gel ou d'une pâte comprenant de 0,05 à 10 % v/v d'un agent reminéralisant et 0,05 à 20 % v/v d'un agent bioadhésif sur la base du volume total de la composition de traitement oral et (ii) un applicateur destiné à appliquer la composition de traitement oral non-abrasive en une couche sur la gencive et les dents de la personne, l'applicateur comprenant à une extrémité un embout constitué d'un matériau flexible, non-poreux, non-absorbant sous la forme d'une feuille planaire ou formant une feuille planaire lorsqu'il est en utilisation.

2. Kit selon la revendication 1, dans lequel l'embout de l'applicateur est sous la forme d'un trapèze.

3. Kit selon la revendication 1 ou 2, dans lequel l'embout de l'applicateur a entre 3 et 3000 protubérances flexibles par cm carré.

4. Kit selon la revendication 3, dans lequel les protubérances flexibles sont sous la forme de feuilles parallèles qui en utilisation deviennent des lamelles en chevauchement et forment une feuille planaire.

5. Kit selon la revendication 3, dans lequel les protubérances sont de forme allongée, comme des poils, qui en utilisation forment une feuille planaire.

6. Kit selon les revendications 2 à 5, dans lequel l'applicateur comprend en outre, adjacent à l'embout, un élément de préhension qui, en utilisation, doit être maintenu par un utilisateur.

7. Kit selon la revendication 6, moyennant quoi l'élément de préhension est de forme allongée.

8. Kit selon les revendications 1 à 7, dans lequel la composition de traitement oral non-abrasive comprend en outre un agent de contrôle microbien.

9. Kit selon les revendications 1 à 8, dans lequel la composition de traitement oral non-abrasive peut être avalée sans danger.
